# EUROPEAN PATENT APPLICATION

(11) **EP 1 939 206 A1**
(43) Date of publication of application: **02.07.2008**
(21) Application number: 06126961.9
(22) Date of filing: 22.12.2006
(51) Int. Cl.: C07D 498/06

(54) **Process for the preparation of an antibacterial quinolone compound**

(71) Applicant: Farmaprojects, S.A., 08902 L'Hospitalet de Llobregat (Barcelona) (ES)
(72) Inventor: Puig Torres, Salvador, 08005, Barcelona (ES); Bessa Bellmunt, Jordi, 08004, Barcelona (ES)
(74) Representative: Barlocci, Anna

(57) **Abstract**

It comprises a process for the preparation of levofloxacin based on a cyclisation reaction of a compound of formula (IV), which has the alcohol group protected, followed by a deprotection reaction and the conversion of the compound obtained to levofloxacin by a process comprising a hydrolysis reaction and a second cyclisation reaction. It also comprises new intermediates compounds.

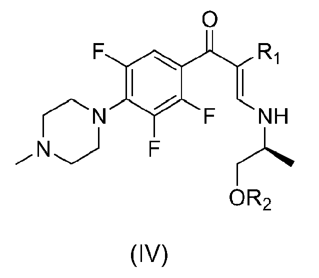

## Description

### FIELD OF THE INVENTION

The present invention relates to a process for preparing levofloxacin. It also relates to new intermediates and their preparation processes.

### BACKGROUND OF THE INVENTION

Levofloxacin is an antibacterial agent with bactericide action. It acts by inhibiting the bacterial DNA-DNA-gyrase complex (topoisomerase II and IV) thus blocking the DNA replication process. It has an extremely wide antibacterial spectrum and acts on both aerobic and anaerobic Gram-positive and Gram-negative bacteria.

Levofloxacin was developed by Daiichi, approved by the FDA on December 20^{th}, 1996 and marketed in the hemihydrate form. Levofloxacin has the following formula:

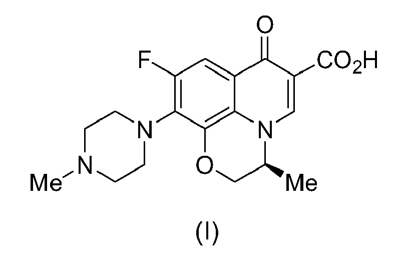

Its chemical name is (S)-9-Fluoro-2,3-dihydro-3-methyl-10-(4-methyl-1-piperazinyl)-7-oxo-7H-pyrido-(1,2,3-de)-1,4-benzoxazine-6-carboxylic acid. Levofloxacin was disclosed for the first time by Daiichi Pharmaceutical Co in EP 206 283. Levofloxacin is the most active enantiomer of ofloxacin, which was disclosed for the first time by Daiichi in EP 047 005.

In EP 206 283 the applicant discloses a synthesis wherein the chirality is introduced by optical resolution or by enatioselective enzymatic hydrolysis of one intermediate. This way of introducing chirality is difficult and costly to scale-up because at least 50% of the intermediate is lost and the reagents for such resolution are expensive.

The prior-art discloses different processes for the synthesis of levofloxacin. In some of these processes, especially the process described in EP 206 283, 1-methylpiperazine is introduced in one of the last steps of the synthesis. These processes produce several by-products and therefore purity and yields are low. Moreover, the solvent used for such introduction is dimethylsulfoxide which should be avoided on industrial scale production. However a person skilled in the art would suppose that the introduction of the 1-methylpiperazine in one of the first steps of the synthesis would cause a deactivation of the ring and therefore drastic conditions would be needed to perform the different cyclisation steps.

In the Korean patent application 10-1999-0034794 the applicant discloses the following route of synthesis:

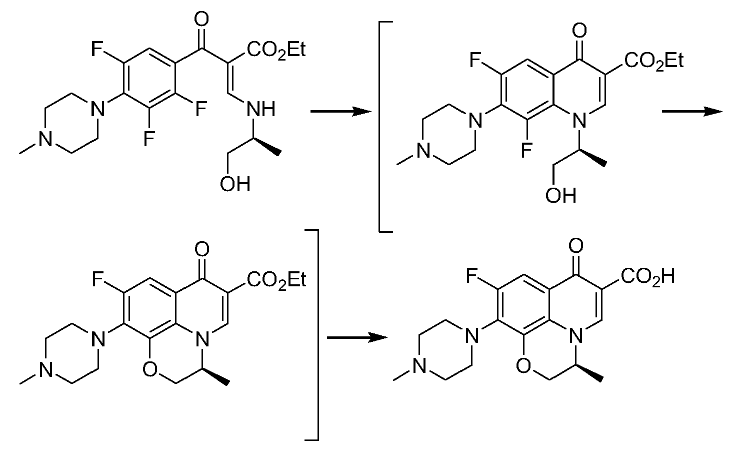

This route of synthesis avoids the introduction of 1-methyl-piperazine in the last step of the synthesis and the chirality is obtained in an efficient way (by using enantiomerically pure and commercially available L-alaninol). In spite of its advantages, this route of synthesis has some drawbacks which make the process difficult on an industrial scale. One of these drawbacks is the use of 2.5 equivalents of NaH as a base (see examples 2 and 3 of the Korean patent application 10-1999-0034794). The use of NaH on an industrial scale is not recommended since it is highly flammable, reacts with water or other protic solvents to form hydrogen and has to be kept in very special conditions. Although the inventors of the present patent application have tried to reproduce the experiments using different bases suitable for industrial scale, such as K₂CO₃, the results were disappointing because of the presence of high amounts of impurities and a low yield. An additional disadvantage of the process described in the foresaid Korean patent application is the use of dioxane in the last step. Dioxane is not a suitable solvent for industrial use due to its toxicity and the fact that it is difficult to remove from the reaction media. Moreover, when NaH is used, the dioxane has to be anhydrous, which further complicates the industrial process.

Despite the teaching of all these prior art documents, the research of new preparation processes of levofloxacin is still an active field due to the importance of levofloxacin and since the industrial exploitation of known processes is difficult, as it has been pointed out in the above-cited documents. Thus, the provision of new preparation processes of levofloxacin is desirable.

### SUMMARY OF THE INVENTION

The problem to be solved by the present invention is to provide an efficient alternative process for preparing levofloxacin and its intermediates.

A first aspect of the invention relates to a process for the preparation of levofloxacin of formula (I), or a pharmaceutically acceptable salt thereof, or a solvate thereof, including a hydrate thereof, 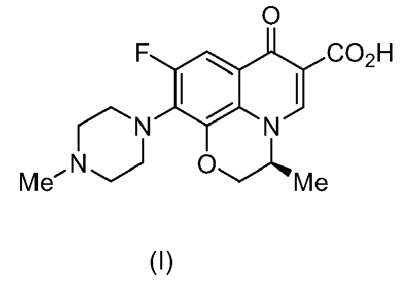 comprising the following steps:
i) the cyclisation of a compound of formula (IV), 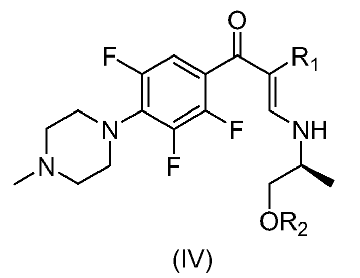 wherein: R₁ is a radical selected from the group consisting of -CO₂R, -CN and CONR'R"; R is a (C₁-C₆)-alkyl radical; R' and R" are independently selected from hydrogen or a (C₁-C₆)-alkyl radical; and R₂ is an alcohol protecting group; to obtain a compound of formula (V), 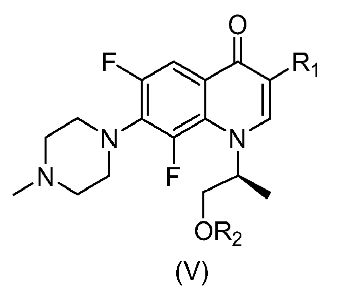 wherein: R₁ and R₂ are as defined above;
ii) the cleavage of the alcohol protecting group of the compound of formula (V) to obtain the compound of formula (VI), 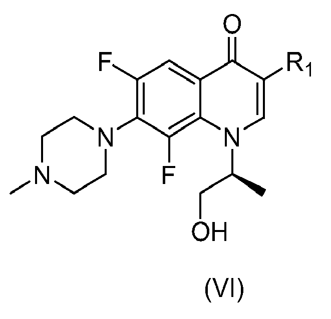 wherein: R₁ is as defined above,
iii) the submission of the compound of formula (VI) either first to a hydrolysis reaction and then to a cyclisation reaction, or, alternatively, either first to a cyclisation reaction and then to a hydrolysis reaction; and optionally the isolation of the resulting levofloxacin as a hydrate or the conversion of the resulting levofloxacin into a hydrate, or into a pharmaceutically acceptable salt, or the conversion of the resulting salt of levofloxacin into a free acid form of levofloxacin, or the conversion of a resulting salt of levofloxacin into a different salt.

One key feature of this process is that the alcohol group is protected during the first cyclisation step. This has surprisingly reduced the formation of by-products and provided higher yields to the process. Furthermore, the use of very strong bases, which are usually difficult to handle on an industrial scale, to perform this reaction is not necessary. The reaction could be performed in the presence or the absence of a base.

In a particular embodiment of the invention, the levofloxacin or its pharmaceutically acceptable salts, or its solvates, including hydrates are obtained submitting the compound of formula (VI) first to a hydrolysis reaction and then to a cyclisation reaction. The hydrolysis reaction gives the compound of formula (VII) or a salt thereof.

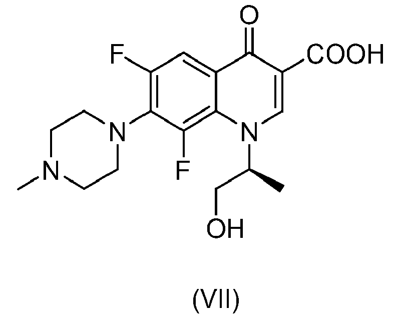

The preparation of levofloxacin or its pharmaceutically acceptable salts, or its solvates, including hydrates from the compound of formula (VII) also forms part of the present invention.This second cyclisation could be performed in mild conditions, with a high yield and a good impurity profile. Obviously these features are very important for the synthesis of active pharmaceutical ingredients on an industrial scale.

Another aspect of the invention relates to the novel compound of formula (VII).

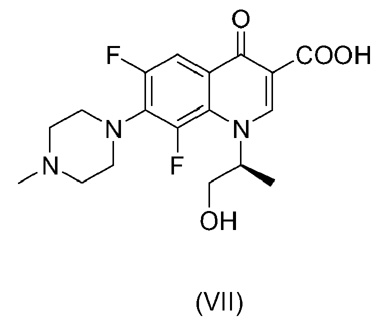

The inventors have isolated and characterised the compound of formula (VII) and surprisingly have found that this compound has very interesting characteristics such as that it is stable, that it is obtained as a solid and that it can be easily purified, for example, by extraction, by leaching, by recrystallisation and even by crystallisation of its potassium salt.

Another aspect of the invention relates to the use of a compound of formula (VII), as defined above, for the synthesis of levofloxacin.

Another aspect of the invention relates to the use of a compound of formula (IV). 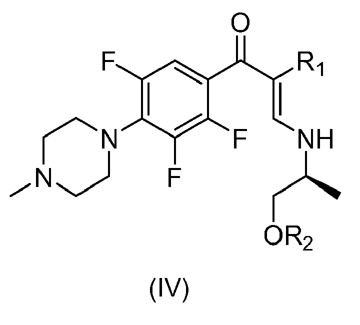 wherein: R₁ and R₂ are as defined above, for the synthesis of levofloxacin.

### Definitions

In the present invention an alcohol protecting group is understood as being any protective group of an alcohol such as esters, ethers, acetals, carbamates or silyl ethers described, for example, by Greene, T. W. et al. in "Protective groups in organic synthesis", John Wiley and Sons, Third Edition, New York, 1999, hereby incorporated by reference.

Unless otherwise indicated from now on, levofloxacin refers to levofloxacin anhydrous, hydrates (preferably hemihydrate and monohydrate) and its pharmaceutical acceptable salts.

The compound of formula (IV). 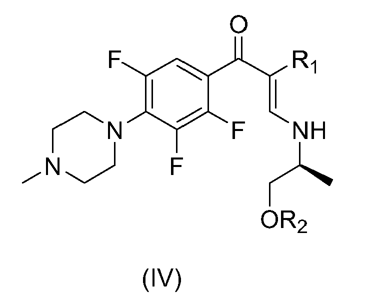 wherein: R₁ is a radical selected from the group consisting of -CO₂R, -CN and CONR'R"; R is a (C₁-C₆)-alkyl radical; R' and R" are independently selected from hydrogen or a (C₁-C₆)-alkyl radical; and R₂ is an alcohol protecting group, refers to the compound of formula (IV) wherein the double bond has a cis configuration, a *trans* configuration or mixtures thereof.

A one pot reaction, synthesis or process refers to reactions which are performed without the isolation of the intermediates.

### DETAILED DESCRIPTION OF THE INVENTION

As it has been mentioned above, the process for the preparation of levofloxacin of the present invention is based on the cyclisation of compounds of formula (IV) to obtain compounds of formula (V), and then the alcohol protecting group is cleaved to obtain compounds of formula (VI). Finally the compound of formula (VI) is converted into levofloxacin. The inventors have surprisingly discovered that when R₂ is not H, but an alcohol protecting group, the yields are higher and the impurity profile is better. The inventors have found that when the reaction is performed without the foresaid protecting group, although the desired product of formula (VI) is obtained, the yield and quality are poor due to the presence of high amounts of impurities. The addition of two further steps in the route of synthesis (protection and deprotection) would usually be avoided by a person skilled in the art because there is usually a loss of product and an increase in the amount of impurities. The present inventors have surprisingly found that these two additional steps do not decrease the total yield and do not increase the amount of impurities, rather that they increase the yield and decrease the total amount of impurities.

In a preferred embodiment this cyclisation could be performed in at least one organic solvent and in the presence of at least one base. The reaction could proceed typically at a temperature of between 0° C and reflux temperature.

In a further preferred embodiment, the base is an organic base or a base selected from the group consisting of a carbonate, a hydride, a hydroxide, and a (C₁-C₆)-alkoxide, of an alkaline or alkaline earth metal. More preferably the base is Na₂CO₃, K₂CO₃, ^{t}BuOK, triethylamine or 1,8-diazabicyclo[5.4.0]undec-7-ene (DBU).

In another embodiment, this reaction could be performed in non polar solvents, such as toluene, optionally using a catalytic amount of a phase transfer agent such as tetrabutyl ammonium bromide, or in polar solvents such as acetonitrile and N,N-dimethylformamide or mixtures thereof. Some advantages of using such solvents are their relatively low toxicity, their acceptability for use on an industrial scale and their low reactivity in the reaction conditions.

In a preferred embodiment the alcohol protecting groups are acyl groups, and more preferably acetyl. The reagents needed to introduce an acyl protecting group are suitable for industrial scale (e.g. are commercially available, allow an easy protection and deprotection). Also the acyl protecting groups are stable in the reaction conditions.

In a further preferred embodiment the cleavage of the acyl group is performed in a (C₁-C₆)-alcohol and in the presence of at least one base. This base could be present only in a catalytic amount and heating is not necessary. These are very mild conditions and easy to scale-up.

Preferably, the process is carried out using a compound of formula (IV) where R₁ is -CO₂R, being R a (C₁-C₆)-alkyl radical. More preferably R is ethyl.

In a preferred embodiment the compounds of formula (IV) are obtained by reacting a compound of formula (III) 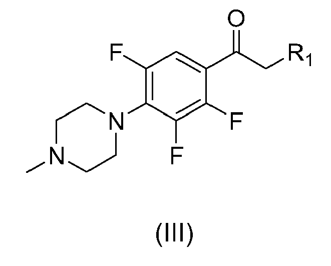 wherein: R₁ is as defined above, first either with dimethyl acetal of N,N-dimethylformamide or alternative with a mixture of ethyl orthoformiate and acetic anhydride, and then by reaction with L-alaninol, followed by submission of the compound obtained to a protection reaction with a suitable reagent to introduce R₂ as an alcohol protecting group.

In a further preferred embodiment the compound of formula (III) is obtained by reaction of a compound of formula (II), 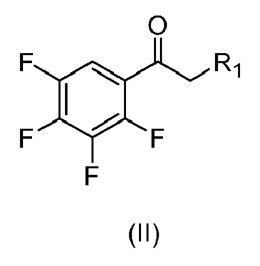 wherein: R₁ is as defined above, with 1-methylpiperazine.

In a preferred embodiment of the first aspect of the invention, in compounds (II), (III), (IV), (V), and (VI), R₁ is -CO₂R, being R a (C₁-C₆)-alkyl radical. Preferably, R is methyl, ethyl or propyl, and more preferably, R is ethyl.This is especially advantageous because ester groups are easier to hydrolyze than cyano or amide groups, to obtain the carboxylic acid present in levofloxacin. In a more preferred embodiment R is ethyl, because the compound of formula (II) is commercially available. Moreover the compound of formula (VI) wherein R is ethyl is obtained as a stable solid, and therefore it is easy to isolate and purify (for example by recrystallisation or leaching). In other embodiments, R could be methyl or propyl.

Additionally in another embodiment the inventors have found that the processes from the intermediate (II) to the compound of formula (VI) could be performed in a "one pot" synthesis, without isolating any intermediate. Thus the industrial scale process is faster, economically advantageous and environmentally friendly (because the amount of organic solvent is reduced). The preferred solvent for the one pot reaction is a non polar solvent such as toluene, although the reaction could be performed as well in polar solvents or mixtures thereof.

As it has been mentioned above, the cyclisation of the compound of formula (VII) or a salt thereof to yield levofloxacin, or its pharmaceutically acceptable salts, or its solvates, including hydrates also forms part of the invention. Compound of formula (VII) is a new intermediate useful for the preparation of levofloxacin.

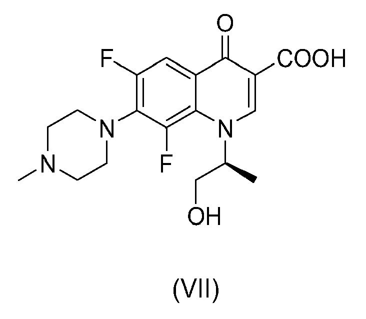

The reaction could proceed typically at a temperature between 0° C and reflux temperature. In a preferrred embodiment the cyclisation takes place in the presence of at least one base and in an appropriate solvent system selected from at least one organic solvent and a mixture of at least one organic solvent and water. In a further preferred embodiment the base is an organic base or a base selected from the group consisting of a hydroxide, an oxide, a carbonate, and a (C₁-C₆)-alkoxide, of an alkaline or alkaline earth metal. More preferably the base is NaOH, Na₂CO₃, KOH, K₂CO₃, NaOEt, KOEt, ^{t}BuOK, triethylamine or DBU.

In a preferred embodiment the solvent is a (C₁-C₆)-alcohol. The inventors have found that is possible to crystallise levofloxacin in the reaction medium where the cyclisation takes places. To achieve this crystallisation the solvent where the cyclisation takes places should have certain characteristics. The solvent, or solvent mixture, should dissolve the compound of formula (VII) and levofloxacin at reflux temperature, but should not dissolve levofloxacin at a lower temperature. (C₁-C₆)-alcohols are among the solvents having these characteristics, and particularly mixtures of at least one (C₁-C₆)-alcohol and water. These mixtures are also important because, by controlling the amount of water, it is possible to control which hydrate form is crystallised (e.g. anhydrous, hemihydrate or monohydrate) and levofloxacin free from salts can be obtained. If the amount of water in the mixture is increased, then, levofloxacin is less soluble at lower temperature, the possibilities to obtain the monohydrate increase and the amount of salts present in the final product is reduced.

Compound of formula (VII) is obtained by hydrolysis of the compound of formula (VI). 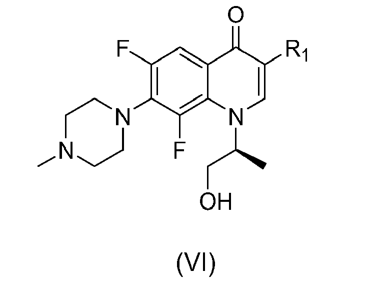 wherein: R₁ is as defined above. In a further preferred embodiment the group R₁ is -CO₂R being R a (C₁-C₆)-alkyl radical, because esters are easy to hydrolyse. More preferably R is methyl, ethyl or propyl. If R is ethyl, then the compound of formula (VI) is obtained as a solid. An amide or a cyano group could also be hydrolysed to obtain the corresponding acid group by following methods well known in the art as described in Advanced Organic Chemistry, Reactions, Mechanisms and Structure. Fourth Edition. Jerry March. Wiley Interscience. Pages 383 and 887.

In a further preferred embodiment the hydrolysis is performed in at least an organic solvent or in mixtures of at least one organic solvent and water and in the presence of at least one base. More preferably the solvent is a (C₁-C₆)-alcohol. In a further preferred embodiment the base is an oxide, a hydroxide, a carbonate or a (C₁-C₆)-alkoxide of a metal or an organic base.

In a further preferred embodiment the two synthetic steps, from the compound of formula (VI) to levofloxacin, are performed in one pot without the isolation of the compound of formula (VII) (without reducing the quality of the levofloxacin obtained). More preferably the solvent used is an organic solvent, mixtures of organic solvents or a mixture of an organic solvent and water. More preferably the solvent is a (C₁-C₆)-alcohol.

Levofloxacin could be purified using methods known in the art such as chromatography or recrystallisation using an organic solvent such as methanol, ethanol, propanol, acetone, isopropanol, dimethylformamide, water or mixtures thereof. It is also possible to control the precipitation of the desired hydrate (especially its hemihydrate and its monohydrate) by using the solvents mentioned above or other organic solvents or mixture thereof, and particularly controlling the amount of water. These techniques are well-known in the art.

Levofloxacin could be formulated as a tablet blending it with HPMC and crospovidone in a planetary mixer and granulating the blend with water. After drying and sieving the granulate, microcrystalline cellulose and magnesium stearyl fumarate are added to it. Finally the tablets are formed by compression and they could be further coated.

Levofloxacin could also be formulated as infusion dissolving it in a solution of NaCl in water and adjusting the pH between 4.0 and 5.5 using HCl_{(aq)} and NaOH_{(aq)}.

The following examples are to illustrate the invention and not to limit its scope in any way.

### EXAMPLE 1

### ethyl 3-oxo-3-(2,3,5-trifluoro-4-(4-methylpiperazin-1-yl)phenyl)propanoate (compound of formula (III) where R₁ is COOEt)

To a solution of 12 g (45.5 mmol) of ethyl 3-oxo-3-(2,3,4,5-tetrafluorophenyl) propanoate in 100 mL of THF, 10 mL (90.1 mmol) of 1-methylpiperazine were added and the mixture heated at reflux for 2 hours. The solvent was distilled under vacuum and the residue was extracted with ethyl acetate and water. The organic phases were distilled under vacuum to obtain 14.9 g of the title compound as an oil in 95 % yield.

RMN ¹H (CDCl₃), δ(ppm) Tautomers keto-enol: 1.23 (t, 3H); 2.30 (3H, s); 2.48 (4H, m, piperazine); 3.36 (4H, m, piperazine); 3.86 (2H, d); 4.20 (2H, q); 5.8 (1 H, s); 7.30-7.50 (1 H, m).

### EXAMPLE 2

### (S)-ethyl 3-(1-hydroxypropan-2-ylamino)-2-(2,3,5-trifluoro-4-(4-methylpiperazin-1-yl)benzoyl)acrylate (compound of formula (IV) where R₁ is COOEt and R₂ is H)

To a solution of 14.9 g (43.3 mmol) of ethyl 3-oxo-3-(2,3,5-trifluoro-4-(4-methylpiperazin-1-yl)phenyl)propanoate in 100 mL of toluene, 10 mL (75.3 mmol ) of dimethyl acetal of N,N-dimethylformamide were added and the mixture heated at reflux for 2 and a half hours. The crude reaction mixture was cooled to room temperature, washed twice with a solution of sodium bicarbonate and the organic phase was distilled under vacuum to dryness. The resultant crude containing ethyl 2-(3,5-difluoro-4-(4-methylpiperazin-1-yl)benzoyl)-3-(dimethylamino)acrylate was dissolved in 150 mL of ethanol, cooled to 15-20°C and 3.6 mL (45.2 mol) of L-alaninol were added. After 1 and a half hours, the ethanol was evaporated under vacuum and extracted with ethyl acetate and washed with sodium bicarbonate solution to give 17.8 g of the title compound as an oil in 96 % yield.

RMN ¹H (CDCl3), δ(ppm) Cis and Trans isomers: 0.9-1.1 (3H, t); 1.36 (3H, d); 2.3 (3H, s); 2.5 (4H, m, piperazine); 3.3 (4H, m, piperazine); 3.5-3.7 (3H, m); 4.0-4.1 (2H, q); 6.8 (1 H, m); 8.2 (1 H, d).

### EXAMPLE 3

### (S)-ethyl 3-(1-acetoxypropan-2-ylamino)-2-(2,3,5-trifluoro-4-(4-methylpiperazin-1-yl)benzoyl)acrylate (compound of formula (IV) where R₁ is COOEt and R₂ is CH₃CO)

15.3 g (35.6 mol) of the oil obtained in EXAMPLE 2 were dissolved in 150 mL of dichloromethane and after adding 6.6 mL (47.3 mmol) of triethylamine the mixture was cooled to 0-5 °C and 4.4 mL (61.8 mmol) of acetyl chloride were added over 30 minutes. The reaction was left at 5-10 °C for 1 and a half hours, and then the solvent was evaporated under vacuum to obtain an oil in almost quantitative yield.

RMN ¹H (CDCl₃), δ(ppm) Cis and Trans isomers: 0.9-1.1 (3H, t); 1.4 (3H, d); 2.1 (3H, s); 2.35 (3H, s); 2.55 (4H, m, piperazine); 3.3 (4H, m, piperazine); 3.7 (1 H, m); 4.0-4.1 (4H, m); 6.9 (1 H, m); 8.2 (1 H, d).

### EXAMPLE 4

### (S)-ethyl 1-(1-acetoxypropan-2-yl)-6,8-difluoro-7-(4-methylpiperazin-1-yl)-4-oxo-1,4-dihydroquinoline-3-carboxylate (compound of formula (V) where R₁ is COOEt and R₂ is CH₃CO)

16.8 g (35.6 mmol) of the oil obtained in EXAMPLE 3 were dissolved in 160 mL of acetonitrile, 17 g (123 mol) of anhydrous potassium carbonate were added and the mixture was heated at reflux for three hours. After the reflux, the salts were filtered and washed with more acetonitrile and the solvent was evaporated under vacuum to give 15.1 g of a dark oil in an estimated 94 % yield.

RMN ¹H (CDCl₃), δ (ppm): 1.37 (3H, t); 1.63 (3H, d); 1.96 (3H, s); 2.32 (3H, s); 2.5 (4H, m, piperazine); 3.3 (4H, m, piperazine); 3.8-4.4 (3H, m); 7.9 (1 H, dd); 8.53 (1 H, s).

### EXAMPLE 5

### (S)-ethyl 6,8-difluoro-1-(1-hydroxypropan-2-yl)-7-(4-methylpiperazin-1-yl)-4-oxo-1,4-dihydroquinoline-3-carboxylate (compound of formula (VI) where R₁ is COOEt)

A catalytic amount of 0.1 g (0.7 mmol) of potassium carbonate was added to a solution of 15.1 g (33.5 mol) of the oil obtained in EXAMPLE 4 in 100 mL of ethanol and the reaction was left stirring at room temperature for 1 hour. The reaction mixture was neutralised with 0.1 mL of acetic acid, cooled to 0-5°C and filtered to obtain 9.0 g (65.6%) of the title compound.

Melting point: 195 - 197 °C.
IR (cm⁻¹): 3316, 1724, 1696,1610,1478, 1235.
RMN ¹H (CDCl₃), δ(ppm): 1.4 (3H, t); 1.67 (3H, d); 2.38 (3H, s); 2.57 (4H, m, piperazine); 3.37 (4H, m, piperazine); 3.8 (2H, m); 4.3 (2H, q); 5.33 (1 H, s); 7.03 (1 H, dd); 8.60 (1 H, s).

### EXAMPLE 6

### (S)-ethyl 6,8-difluoro-1-(1-hydroxypropan-2-yl)-7-(4-methylpiperazin-1-yl)-4-oxo-1,4-dihydroquinoline-3-carboxylate (compound of formula (VI) where R₁ is COOEt)

Alternatively, the title compound could be obtained in a one pot process starting from 3-oxo-3-(2,3,4,5-tetrafluorophenyl)propanoate:

To a solution of 62 g (0.235 mol) of 3-oxo-3-(2,3,4,5-tetrafluorophenyl)propanoate in 450 mL of toluene, 68 mL (0.61 mol) of 1-methylpiperazine were added and the mixture heated at 70 °C for 80 minutes. The solution was cooled to room temperature and washed twice with a solution of water with sodium bicarbonate. The organic phase was dried by azeotropic distillation of toluene and the initial volume was recovered with more toluene. Dimethyl acetal of N,N-dimethylformamide 40 mL (0.30 mol) were added and the resultant mixture was heated at reflux for 2 and a half hours. The crude reaction mixture was cooled and washed twice with a solution of sodium bicarbonate, the organic phase was dried by azeotropic distillation and the original volume was recovered with more toluene. 19 mL of L- alaninol (0.24 mol) were added to the cooled solution at 15-20 °C and after stirring the reaction for 1 and a half hours, the mixture was washed twice with a solution of sodium bicarbonate, the organic phase was dried by azeotropic distillation and the original volume was recovered with more toluene. 56 mL (0.40 mol) of triethylamine were added and the mixture was cooled to 0-5 °C then 31 mL (0.43 mol) of acetyl chloride were added over 30 minutes. The reaction was left at 5-10 °C for 1 and a half hours, the crude reaction mixture was washed twice with a solution of sodium bicarbonate, the organic phase was dried by azeotropic distillation and the original volume was recovered with more toluene. 120 g (0.87 mol) of potassium carbonate and 1 g (0.003 mol) of tetrabuthylammonium bromide were added and the mixture was heated at reflux for three hours. The reaction was cooled to room temperature, filtered and the solid was washed with more toluene. The toluene was distilled under vacuum and stripped with the addition of methanol. 250 mL of methanol and 1 g (0.007 mol) of potassium carbonate were added and the mixture was left stirring for 1 hour. The potassium carbonate was neutralised with 1 mL of acetic acid and the solvent was evaporated under vacuum and stripped with ethyl acetate until a precipitate was formed. 300 mL of ethyl acetate were added and after stirring for 1 hour, the suspension was cooled at 5-10 °C for 2 hours then the solid was filtered and washed with cold ethyl acetate. 53 g of (S)-ethyl 6,8-difluoro-1-(1-hydroxypropan-2-yl)-7-(4-methylpiperazin-1-yl)-4-oxo-1,4-dihydroquinoline-3-carboxylate were obtained, and a second crop of 7 g was recovered after concentrating the filtrate to give a total of 61 g (63.5 % yield for the six steps).

### EXAMPLE 7

### (S)-ethyl 6,8-difluoro-1-(1-hydroxypropan-2-yl)-7-(4-methylpiperazin-1-yl)-4-oxo-1,4-dihydroquinoline-3-carboxylate (compound of formula (VI) where R₁ is COOEt)

1 g (0.0023 mol) of the oil obtained following EXAMPLE 2 was dissolved in 25 mL of acetonitrile. 1 g (0.007 mol) of potassium carbonate was added and the mixture was heated at reflux for three hours. After the reflux, the salts were filtered and washed with more acetonitrile and the solvent was evaporated under vacuum and stripped with ethyl acetate until a precipitate was formed. 7 mL of ethyl acetate were added and after stirring for 1 hour, the suspension was cooled at 5-10 °C for 2 hours and the solid was filtered and washed with cold ethyl acetate to obtain 0.4 g (43%) of title compound, showing several impurities on the TLC.

### EXAMPLE 8

### (S)-6,8-difluoro-1-(1-hydroxypropan-2-yl)-7-(4-methylpiperazin-1-yl)-4-oxo-1,4-dihydroquinoline-3-carboxylic acid (compound of formula (VII))

1.7 g (30 mmol) of potassium hydroxide were dissolved in 100 mL of ethanol 96 %. At room temperature, 5.0 g (12.2 mmol) of (S)-ethyl 6,8-difluoro-1-(1-hydroxypropan-2-yl)-7-(4-methylpiperazin-1-yl)-4-oxo-1,4-dihydroquinoline-3-carboxylate were added and the solution was kept at room temperature for 3 hours. The potassium hydroxide was neutralised with 1.6 mL of glacial acetic acid and the solution was cooled at 0 °C for 1 hour to give a precipitate which was filtered and washed with cold ethanol 96 % to obtain 2.6 g (55.8%) of the title compound.

Melting point: 250-255°C
IR (cm-¹): 2941, 1619, 1399
RMN ¹H (CDCl3), δ(ppm): 1.61 (3H, d); 2.35 (3H, s); 2.59 (4H, m, piperazine); 3.39 (4H, m, piperazine); 3.9 (2H, m); 5.33 (1 H, s); 7.72 (1 H, d); 8.80 (1 H, s).

In an additional experiment, the potassium salt of the title compound was also isolated by a simple filtration of the reaction mixture before neutralising with acetic acid.

### EXAMPLE 9

### (S)-9-Fluoro-2,3-dihydro-3-methyl-10-(4-methyl-1-piperazinyl)-7-oxo-7H-pyrido(1,2,3-de)-1,4-benzoxazine-6-carboxylic acid (levofloxacin)

2.5 g (6.5 mmol) of (S)-6,8-difluoro-1-(1-hydroxypropan-2-yl)-7-(4-methylpiperazin-1-yl)-4-oxo-1,4-dihydroquinoline-3-carboxylic acid were added to a solution of 1.3 g (23 mmol) of potassium hydroxide in 20 mL of ethanol 96% and heated at reflux for 3 hours. 1.25 mL of acetic acid were added to the mixture at room temperature and after cooling to 0-5°C it was filtered to obtain 1.8 g (76%) of levofloxacin hemihydrate.

### EXAMPLE 10

### (S)-9-Fluoro-2,3-dihydro-3-methyl-10-(4-methyl-1-piperazinyl)-7-oxo-7H-pyrido(1,2,3-de)-1,4-benzoxazine-6-carboxylic acid (levofloxacin)

Alternatively, the title compound could be obtained in a one pot process starting from (S)-ethyl 6,8-difluoro-1-(1-hydroxypropan-2-yl)-7-(4-methylpiperazin-1-yl)-4-oxo-1,4-dihydroquinoline-3-carboxylate:

3.0 g (7.3 mmol) of (S)-ethyl 6,8-difluoro-1-(1-hydroxypropan-2-yl)-7-(4-methylpiperazin-1-yl)-4-oxo-1,4-dihydroquinoline-3-carboxylate were added to a solution of 1.56 g (27.8 mmol) of potassium hydroxide in 80 mL of ethanol 96 %. After hydrolysis of the ester at room temperature, the reaction mixture was heated at reflux for 2 hours and a half. The reaction was cooled to room temperature and neutralised, with approximately 1.5 mL of glacial acetic acid, to pH 7. After neutralisation, 10 mL of water were added to the mixture and then it was cooled at 0-5 °C for 2 hours and finally filtered to obtain 2.1 g (78%) of levofloxacin hemihydrate.

An alternative method used for the isolation of levofloxacin was by evaporation of the ethanol under vacuum and addition of 15 mL of water, the mixture was stirred at 5 °C and filtered to give an hydrated levofloxacin in almost quantitative yield which was washed with a small amount of ethanol and crystallised from isopropyl alcohol to give pure levofloxacin hemihydrate in 77% yield.

## Claims

1. A process for the preparation of levofloxacin of formula (I), or a pharmaceutically acceptable salt thereof, or a solvate thereof, including a hydrate, 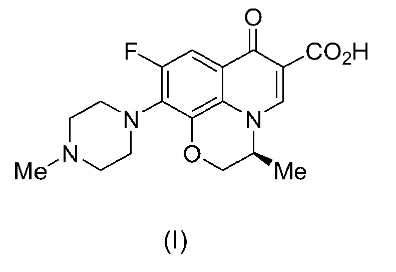 comprising the following steps:
i) the cyclisation of a compound of formula (IV), 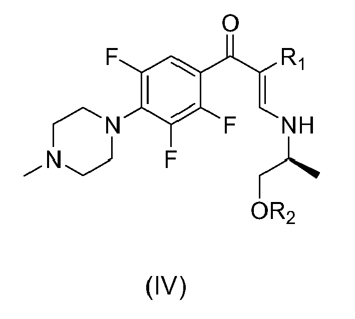 wherein:
R₁ is a radical selected from the group consisting of -CO₂R, -CN and -CONR'R";
R is a (C₁-C₆)-alkyl radical;
R' and R" are independently selected from hydrogen or a (C₁-C₆)-alkyl radical; and
R₂ is an alcohol protecting group;
to obtain a compound of formula (V), 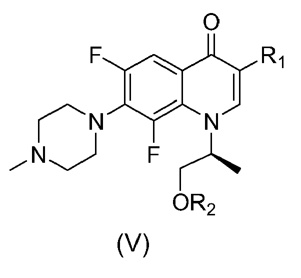 wherein R₁ and R₂ are as defined above;
ii) the cleavage of the alcohol protecting group of compound (V) to obtain the compound of formula (VI), 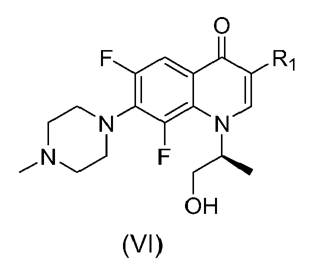 wherein R₁ is as defined above;
iii) the submission of the compound of formula (VI) either first to a hydrolysis reaction and then to a cyclisation reaction, or, alternatively, either first to a cyclisation reaction and then to a hydrolysis reaction; and optionally the isolation of the resulting levofloxacin as a hydrate or the conversion of the resulting levofloxacin into a hydrate, or into a pharmaceutically acceptable salt, or the conversion of the resulting salt of levofloxacin into a free acid form of levofloxacin, or the conversion of a resulting salt of levofloxacin into a different salt.

2. The process according to claim 1, wherein the cyclisation is performed in at least one organic solvent in the presence of at least one base.

3. The process according to claim 2, wherein the base is an organic base or a base selected from the group consisting of a carbonate, a hydride, a hydroxide, and a (C₁-C₆)-alkoxide, of an alkaline or an alkaline earth metal.

4. The process according to claim 3, wherein the base is selected from the group consisting of Na₂CO₃, K₂CO₃, NaOH, KOH, ^{t}BuOK, triethylamine and 1,8-diazabicyclo[5.4.0]undec-7-ene.

5. The process according to any of the claims 2 to 4, wherein the solvent is selected from the group consisting of acetonitrile, toluene, dimethylformamide and mixtures thereof.

6. The process according to any of the preceding claims, wherein R₂ is acyl.

7. The process according to claim 6, wherein R₂ is acetyl.

8. The process according to any of the claims 6 to 7, wherein the deprotection reaction to remove the alcohol protecting group is performed in a (C₁-C₆)-alcohol as solvent and in the presence of a base.

9. The process according to any of the preceding claims, wherein the compound of formula (IV) is obtained by reaction of a compound of formula (III), 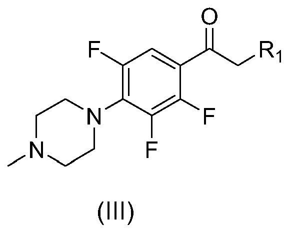 wherein R₁ is as defined in claim 1;
first either with dimethyl acetal of N,N-dimethylformamide or alternatively with a mixture of ethyl orthoformiate and acetic anhydride, and then by reaction with L-alaninol, followed by submission of the compound obtained to a protection reaction with a suitable reagent to introduce R₂ as an alcohol protecting group.

10. The process according to claim 9, wherein the compound of formula (III) is obtained by reaction of a compound of formula (II), 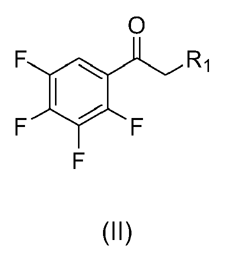 wherein R₁ is as defined in claim 1;
with 1-methylpiperazine.

11. The process according to any of the preceding claims, wherein R₁ is - CO₂R, being R a (C₁-C₆)-alkyl radical.

12. The process according to claim 11, wherein R is ethyl.

13. The process according to any of the claims 1 to 12, wherein the preparation of the compound of formula (VI) is performed in one pot without isolating any intermediate.

14. A process for the preparation of levofloxacin of formula (I), or a pharmaceutically acceptable salt thereof, or a solvate thereof, including a hydrate, 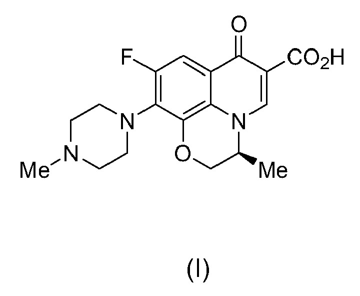 comprising the cyclisation of the compound of formula (VII) or a salt thereof.

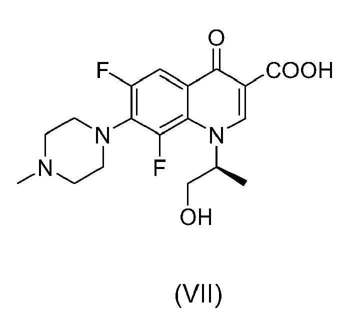

15. The process according to claim 14, wherein the cyclisation is performed in the presence of at least one base and in an appropriate solvent system selected from at least one organic solvent and mixtures of at least one organic solvent and water.

16. The process according to claim 15, wherein the base is an organic base or a base selected from the group consisting of a hydroxide, an oxide, a carbonate and a (C₁-C₆)-alkoxide, of an alkaline or an alkaline earth metal.

17. The process according to claim 16, wherein the base is NaOH, Na₂CO₃, KOH, K₂CO₃, NaOEt, KOEt, ^{t}BuOK, triethylamine or 1,8-diazabicyclo[5.4.0]undec-7-ene.

18. The process according to any of the claims 15 to 17, wherein the organic solvent is a (C₁-C₆)-alcohol.

19. The process according to any of the claims 14 to 18, wherein the compound of formula (VII) is obtained by hydrolysis of the compound of formula (VI), 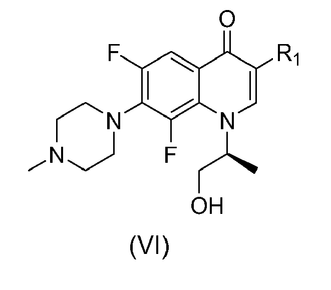 wherein:
R₁ is a radical selected from the group consisting of -CO₂R, -CN and -CONR'R";
R is a (C₁-C₆)-alkyl radical; and
R' and R" are independently selected from hydrogen or a (C₁-C₆)-alkyl radical.

20. The process according to claim 19, wherein R₁ is -CO₂R.

21. The process according to claim 20, wherein R is selected from the group consisting of methyl, ethyl and propyl.

22. The process according to any of the claims 19 to 21, wherein the hydrolysis of R₁ is performed in at least one organic solvent in the presence of at least one base.

23. The process according to claim 22, wherein the solvent is a (C₁-C₆)-alcohol.

24. The process according to any of the claims 22 to 23, wherein the base is an organic base or a base selected from the group consisting of, an oxide, a hydroxide, a carbonate and a (C₁-C₆)-alkoxide, of an alkaline or an alkaline earth metal.

25. The process according to any of the claims 19 to 24, wherein the hydrolysis and later cyclisation are performed in one pot without the isolation of the compound of formula (VII).

26. The process according to any of the claims 1 to 13, wherein the compound of formula (VI) is hydrolysed and cycled following any of the processes claimed in the claims 19 to 24.

27. The process according to any of the claims 19 to 25, wherein the compound of formula (VI) is obtained following any of the processes claimed in any of the claims 1 to 13.

28. The use of a compound of formula (IV) 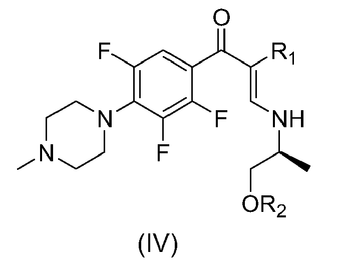 wherein:
R1 is a radical selected from the group consisting of -CO2R, -CN and CONR'R";
R is a (C₁-C₆)-alkyl radical;
R' and R" are independently selected from hydrogen or a (C₁-C₆)-alkyl radical;
and R2 is an alcohol protecting group;
for the synthesis of levofloxacin.

29. A compound of formula (VII). 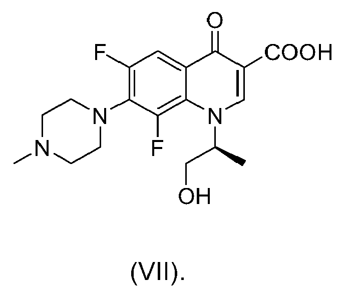 and its salts, in particular its potassium salt.

30. The use of a compound according to claim 29 for the synthesis of levofloxacin.
